# NEUE EUROPÄISCHE PATENTSCHRIFT

(11) **EP 1 347 734 B2**
(45) Veröffentlichungstag und Bekanntmachung des Hinweises auf die Entscheidung über den Einspruch: **29.07.2009**
(45) Hinweis auf die Patenterteilung: 18.01.2006
(21) Anmeldenummer: 01994633.4
(22) Anmeldetag: 02.11.2001
(51) Int. Cl.: A61K 8/894, A61K 8/92, A61Q 17/04, A61Q 19/00

(54) **VESIKELBILDENDE HAUTÖLE ENTHALTEND W/O-EMULGATOREN MIT EINEM HLB-WERT VON 2-5,9, HERSTELLUNGSVERFAHREN UND VERWENDUNG**
VESICLE FORMING SKIN OIL CONTAINING W/O-EMULSIFIERS WITH A HYDROPHILIC-LIPOPHILIC BALANCE OF 2-5.9, METHOD FOR THE PRODUCTION AND USE THEREOF
HUILES POUR LA PEAU FORMANT DES VESICULES ET CONTENANT DES EMULSIFIANTS EAU DANS L'HUILE PRESENTANT UN EQUILIBRE HYDROPHILE-LIPOPHILE EGAL A 2-5,9, LEUR PROCEDE DE PREPARATION ET LEUR UTILISATION

(30) Priorität: 15.12.2000 DE 10062610
(43) Veröffentlichungstag der Anmeldung: 01.10.2003
(73) Patentinhaber: Merz Pharma GmbH & Co. KGaA, 60318 Frankfurt (DE)
(72) Erfinder: PASPALEEVA-KÜHN, Valentina, 60323 Frankfurt (DE); SCHATSCHNEIDER, Simone, 65207 Wiesbaden (DE); BEUTLER, Rolf, D., 64739 Höchst/Hummetroth (DE)
(74) Vertreter: Müller, Claudia
(86) Internationale Anmeldenummer: PCT/EP2001/012709
(87) Internationale Veröffentlichungsnummer: WO 2002/047617

(56) Entgegenhaltungen:
- EP-A- 0 467 218
- EP-A- 0 534 823
- EP-A- 0 557 825
- EP-A- 0 930 064
- EP-A2- 0 930 064
- WO-A1-93//02660
- DE-A1- 4 205 548

## Beschreibung

Die vorliegende Erfindung betrifft fetthaltige Hautöle, enthaltend eine oder mehrere öllösliche Komponenten , einen oder mehrere W/O-Emulgatoren mit einem HLB-Wert von 2-5,9 und ein oder mehrere vesikelbildenden Lipide sowie gegebenenfalls einen oder mehrere Zusatzstoffe, ausgewählt aus etherischen Ölen, Antioxidantien, Parfümstoffen, Konservierungsmitteln, Wirkstoffen, UV-Filtern, Vitaminen. Konsistenzmodulatoren, Lösungsvermittlern , deren Herstellung sowie ihre Verwendung als Hautöl, insbesondere als Hautpflege-, Hautschutz-, Sport-, Massage- oder Sonnenschutzöl. Diese Hautöle können auf trockene und insbesondere auf die nasse Haut aufgetragen werden, wobei sie selbstemulgierend wirken und spontan Liposomen bilden. Somit weisen sie die damit bedingten Vorteile (Wirkstoff-Carrier, Aufbau der Hautbarriere), auchdurch das leichte Einziehen in die Haut auf, ohne auf der Haut einen störenden Fettfilm zu hinterlassen.

Hautöle haben einen festen Platz in der breiten Palette kosmetischer Pflegeprodukte eingenommen. Sie haben die Aufgabe, das Defizit an Hautlipiden auszugleichen, die Haut weich und geschmeidig zu machen, die Hautelastizität zu fördern oder durch die Bildung eines hydrophoben Films die Haut zu schützen. Bei Massagen werden sie als Gleitmittel verwendet.

Durch die Inkorporierung spezifischer Wirkstoffe können sogenannte Hautfunktionsöle, z.B. Sonnenschutzöle, Sportöle formuliert werden.

Es handelt sich um flüssige, ölige Zubereitungen, die durch Mischen aus verschiedenen Fetten und Ölen, Wachsen, Fettsäureestern und flüssigen Kohlenwasserstoffen oder Silikonölen zubereitet werden.

Weiterhin können Stabilisatoren wie Antioxidantien, Konservierungsmittel oder spezifische Wirkstoffe, wie UV-Filter, Vitamine, etherische Öle oder Pflanzenextrakte inkorporiert werden. Sie sogenannten hydrophilen Öle enthalten zusätzlich einen O/W-Emulgator und können mit Wasser durch Bildung einer O/W-Emulsion abgewaschen werden.

Solche üblichen Hautöle sind auch in der Monographie von K.Schrader, Grundlage und Rezepturen der Kosmetika, 2. Aufl. (1989, Hüthig Buchverlag), S. 525-528 beschrieben.

Im "Handbuch der Kosmetika und Riechstoffe", Bd. III, 1973, S. 482-489 (Dr. A. Hüthig Verlag Heidelberg) sind verschiedene Hautöle beschrieben. Als Komponenten können z.B. eingesetzt werden: nichttrocknende Öle, Mineralöle verschiedener Viskosität, Isopropylmyristat und -palmitat, Isopropyloleat desodoriert, Isopropylisostearat, Hexadecylalkohol flüssig, Hexadecylisostearat, Oleylalkohol reinst (HD-Eutanol), Cetiol LG, Cetiol HE, Myritol 318, Cremogen HP, Miglyol 812, Cetiol B, Oleyloleat, Decyloleat (Cetiol V), Perhydrosqualen (Cosbiol), flüssige Acetoglyceride, Amerchol L-101, Acetulan (American Cholesterol), Lanolinderivate (z.B. Lanolin flüssig; Modulan usw.), iso-Hexadecylpalmitostearat (Wickenol 1109-H), Cetylpolypropylenglycolether (P.R.O. Cetyl Alcöhol 30), Lanolinalkoholpolypropylenglycolether (P.R.O. Lanolin Alchol 30), Hexadecylpolypropylenglycolether (P.R.O. Hexadecylalcohol 30), Silikonöle, Propylenglycoldipelargonat (Emery 3771-D).

Als Zusatzstoffe werden insbesondere allgemein die folgenden "Wirkstoffe" beschrieben:
Lecithine, Lanolin flüssig und Lanolinderivate, ölige Drogenextrakte (Kamille, Arnika, Hypericum, Calendula), ölige Hautextrakte (und Drüsenextrakte), Campher, Menthol, Carmpherphenolat, Ichthyol (öllöslich), Aluminiumstearat (auch als Verdickungsmittel), flüssige Polyethylenglycolmonofettsäureester, Vitamine (A, D₃, E usw.), β-Carotin, Chlorophyll, öllöslich.

Insbesondere wird ein Gesichtsöl aus Olivenöl, Eutanol G, Neoba O, Lecithinöl, PurcellinÖl, Parfüm, Farbe, Konservierungsmittel offenbart. Daneben werden verschiedene Massage-, Körper- und Sportöle beschrieben, welche überwiegend Paraffinöl enthalten (vgl. S. 487-488).

Weiterhin werden abwaschbare Hautöle beschrieben, die die o.g. O/W-Emulgatoren aufweisen.

In Schrader, K.H., Grundlagen und Rezepturen der Kosmetika, 1989, S. 525-528, werden Hautöle beschrieben, welche als Ölkomponente Cetiol V sowie Wirkstoffe und Lecithin aufweisen können.

Die DE 31 41 761 beschreibt die Verwendung von Glucosederivaten in z.B. Ölen, wobei die Glucosederivate als oberflächenaktive Stoffe eingesetzt werden.

In SÖFW,115(1989),S.344-350 werden allgemeine Grundlagen zur Herstellung von wasserfreien Produkten offenbart und auch Hautöle beschrieben. Diese sind als lipidhaltige, wasserfreie, flüssige Präparate charakterisiert und können zusätzlich einen O/W-Emulgator mit einem HLB-Wert von größer 8 aufweisen. Als Ölkomponenten werden dabei üblichen Paraffine oder z.B.Octyldodecanol, Myritol 318, Cetiol, Miglyol und weiterhin ggf. flüssiges Lanolin, Phenylmethylsilicon beschrieben. Diese Hautöle sollen insbesondere wasserabweisend sein (vgl.S.345,Pt. 2.1.1.2.6).
In SÖFW,98(1972),S.889-891 werden hydrophile Öle beschrieben, die neben den üblichen Ölkomponenten nichtionogene Emulgatoren wie polyoxyethylierte Laurate/Oleate und entsprechende Sorbitan-Derivate mit einem HLB-Wert von 8-11 aufweisen. Solche polyoxyethylierten Produkte sind bekanntlich O/W-Emulgatoren und sollen O/W-Emulsionen bilden. Hierzu zählen auch Kombinationen von Emulgatoren mit einem Gesamt-HLB-Wert von 8 bis 11, wie z.B. die Kombination aus Arlatone® T (HLB = 9,2), Tween® 85 (HLB = 11,0) und Span® 85 (HLB = 1,8)

In der EP-A 0 467 218 werden Lipidkombinationen beschrieben, welche wenigstens 2 der folgenden Komponenten aufweisen: ungesättigte Fettsäuren und/oder deren Tocopherylester; n-Alkane; Squalen; Cholesterin und/oder Wollwachsalkohol; Triglyceride; Wachsester. Besonders bevorzugt sind Kombinationen aus ungesättigten Fettsäuren/Cholesterin/Wollwachsalkohol. Diese Kombinationen sollen als solche oder in einer wasserhaltigen Grundlage verarbeitet auf der Haut anwendbar sein.

Liposomenhaltige Pflege- und Reinigungsformulierungen sind beschrieben in EP Nr. 0 523 418, EP 0 557 825, DE 198 54 827. Es handelt sich hierbei in der Regel um Hydrogele, O/W-Emulsionen oder Waschprodukte. So beschreibt die EP-B 0 557 825 ölhaltige Bade- und Duschzusätze aus Ölkomponenten, vesikelbildenden Lipiden und O/W-Tensiden mit einem HLB-Wert von 6-13. Diese Mittel sind Reinigungsprodukte. Die EP-B 0 523 418 betrifft liposomenhaltige Cremes mit Öl- und Wasserphase und bestimmten polyoxyethylierten Tensiden als Emulgatoren mit HLB-Werten von 9-13. Diese erreichen allerdings nicht die Pflegewirkung eines Hautöls. Außerdem werden bei Hautpflegeprodukten üblicherweise Vesikel erst durch aufwendige Verfahren hergestellt und in die gewünschte Zubereitung eingearbeitet. Insbesondere bei Emulsionen können Stabilitätsprobleme auftreten.

Die DE 198 54 827 betrifft tensidhaltige Reinigungsmittel, welche ölfrei sind und Sterole als Liposomenbildner aufweisen.

Aufgabe vorliegender Erfindung ist es daher, ölhaltige Produkte bereitzustellen, welche auch auf die nasse Haut aufgetragen werden können, dort selbstemulgierend wirken und gleichzeitig Liposomen bilden, so daß auch ein Pflege-Effekt wie beim Eincremen erzielt wird, ohne dass die Haut zuvor einer Trocknung unterzogen wird und ohne dass der bei Hautölen übliche Fettfilm vorliegt, welcher erst nach längerer Zeit oder ggf. erst durch Abreiben oder Abwaschen entfernt ist bzw.werden kann.

Diese Aufgabe wird erfindungsgemäß gelöst durch Hautöl-Zusammensetzungen, enthaltend eine oder mehrere öllösliche Komponenten und einen oder mehrere W/O-Emulgatoren mit einem HLB-Wert von 2-5,9, und ein oder mehrere vesikelbildenden Lipide sowie gegebenenfalls einen oder mehrere Zusatzstoffe, ausgewählt aus Wirkstoffen, Antioxidantien, Parfümstoffen, Konservierungsmitteln, UV-Filtern, Vitaminen, Farbstoffen, Lösungsvermittlern, Konsistenzmodulatoren mit Ausnahme von Zusammensetzungen, enthaltend Cholesterin und/oder Wollwachsalkohol.

Der WO-Emulgator mit einem HLB-Wert von 2-5,9 ist dabei ausgewählt aus ethoxylierten Fettsäuren, ethoxylierten Triglyceriden, (Poly)glycerylestern, Glucose-Estern, Polysiloxan-Copolymeren, Siloxan-Derivatehaltigen Emulgator-Gemischen, oder Mischungen hiervon.

Bei Kombination von den genannten Ölkomponenten mit den beschriebenen W/O-Emulgatoren und den Vesikeibildnern hat sich gezeigt, dass beim Auftragen, insbesondere auf die nasse Haut das Öl selbstemulgierend wirkt und somit sowohl leicht applizierbar als auch ohne störenden Fettfilm ist. Dies ist überraschend, da hier keine zur Herstellung von Emulsionen üblichen Apparaturen, insbesondere Rührer und Hochleistungs-Homogenisatoren benutzt werden. Ferner bilden sich überraschend Liposomen. Dies konnte nicht erwartet werden, da hier insgesamt nur eine geringe Menge Wasser vorliegt (etwa 1:1 im Verhältnis zum ÖI), während bei analogen Anwendungen in der Dusche oder beim Baden weitaus höhere Mengen an Wasser (etwa die 10-fache bzw. 100-fache bis 1000-fache Menge), bezogen auf das Ölbad, vorhanden sind und Vesikel normalerweise eine solche weitaus höhere Menge an Wasser zur Bildung benötigen.

In der erfindungsgemässen Hautöl-Zusammensetzung sind die öllösliche(n) Komponente(n) in einer Menge von 50-99 %, der oder die W/O-Emulgatoren in einer Menge von 0,5-10 % , das oder die vesikelbildenden Lipide in einer Menge von 0,01-10% und der oder die Zusatzstoffe in einer Menge von 0-40 % , insbesondere 0,1-40%, bevorzugt 0,5-20% und ganz besonders bevorzugt 1-15% insgesamt vorhanden.

Der oder die Emulgatoren können erfindungsgemäß wie erwähnt in Mengen von 0,5-10 %, insbesondere von 1,0-8% und ganz bevorzugt 2-5 % und die Vesikelbildner in einer Menge von insbesondere 0,01-10%, vorzugsweise 0,5-5% und insbesondere 1-3 % vorhanden sein.
Die Ölkomponente/n ist dabei in Mengen von 50-99%, insbesondere 70-97% und besonders von 85-97% vorhanden.
In einer besonders bevorzugten Ausgestaltung können die Zusammensetzungen ein oder mehrere W/O-Emulgatoren (allein oder in Mischung miteinander) mit einem HLB-Wert von insbesondere 3-5,5 und ganz besonders von 3,5-5,5 eingesetzt werden.
Die Mengen entsprechen den oben angegebenen.
In einer weiteren bevorzugten Ausführungsform ist/sind die Ölkomponente(n) in einer Menge von 85-97 %, der/die Emulgatoren in einer Menge von 1-10 %, insbesondere 2-6% und ganz besonders 1-5%, die Vesikelbildner in einer Menge von 0,5-5% und die Zusatzstoffe in einer Menge von 1-20, insbesondere 1-15% vorhanden.

Besonders bevorzugte Emulgatoren sind folgende, wobei der HLB-Wert jeweils in Klammem angegeben ist:

### Polyethoxylierte Produkte

Polyethoxylierte Fettsäuren wie PEG-2 Oleate (HLB = 5,0), PEG-4 -Distearate (HLB = 3,0), PEG-2 Stearate (HLB = 4,4), Ceteareth-3, (Volpo®CS3, HLB = 5,0);

Ethoxylierte Triglyceride wie PEG-5 Castor Oil (HLB = 3,9), PEG-6 Diricinoleate (HLB = 5,0), PEG-7 Hydrogenated Castor Oil (Cremophor®WO 7, HLB = 5,0);

### (Poly)glycerylderivate

Polyglycerylester wie Polyglyceryl-3 Diisostearate (Lameform® TGI, HLB = 3,5), Polyglyceryl-2 Dipolyhydroxystearate (Dehymuls® PGPH, HLB = 3,5), Diisostearoyl Polyglyceryl-3 Diisostearate (Isolan® PDI, HLB ca. 5), Polyglyceryl-3 Oleate (Isolan® GO 33, HLB ca. 5), Polyglyceryl-3 Dioleate (Cremophor® GO 32, HLB ca. 5), Polyglyceryl-4 Isostearate (isolan® GI 34, HLB ca. 5);
Glycerin-Ester wie Glyceryl Ricinoleate (Cithrol® GMR N/E, HLB = 2,7), Glyceryl Laurate (Cithrol® GML N/E, HLB = 4,9), Glyceryl Dioleate S/E (Cithrol® GDO S/E, HLB = 2,9);

### Glucose-Derivate

Glucose Ester wie Methyl Glucose Dioleate (Isolan® DO, HLB ca. 5), Methyl Glucose Isostearate (Isolan® IS, HLB ca. 5);

### Siloxan-Derivate

Polysiloxan-Copolymere, insbesondere die nachstehend genannten aus dieser Gruppe.

Bevorzugt sind ethoxylierte Produkte wie Fettsäuren und Triglyceride wie PEG-2 Oleate, PEG-7 Hydrogenated Castor Oil (Cremophor® WO 7), (Poly)glycerylester sowie Glucose -Ester, und Polysiloxan-Copolymere oder Mischungen hiervon.

Besonders bevorzugt sind Polysiloxan-Copolymere wie Polysiloxan-Polyether-Copolymere, insbesondere Polysiloxan-Polyalkyl-Polyether-Copolymere wie Cetyl Dimethicone Copolyol (Abil® EM 90, HLB ca. 5), Laurylmethicone Copoyol (Dow Corning® Q2-5200, HLB ca. 4)

Weiterhin besonders bevorzugt sind auch Siloxan-Derivate-haltige Emulgatorgemische wie das handelsübliche Abil® WE 09 (HLB ca. 5) bestehend aus Cetyl Dimethicone Copolyol, Polyglyceryl-4 Isostearate und Hexyl Laurate.

Alternativ können auch einzelne Emulgatoren aus jeweils einer der genanntenGruppen oder auch aus verschiedenen Gruppen kombiniert werden, wobei Kombinationen von Polysiloxan-Copolymeren und polyglycerylestern bevorzugt sind.

Als Olkomponente sind übliche, vorzugsweise flüssige Lipide geeignet, diese können einzeln oder im Gemisch vorliegen insbesondere sind die folgenden gruppen und Beispiele hierfür geeignet:

### Kohlenwasserstoffe

Kohlenwasserstoffe wie Squalen, Squalan, insbesondere auch flüssige Paraffine, Isoparaffine, Dioctylcyclohexane (Cetiol®S), Isohexadecane (Arlamo® HD);

**Fettalkohole** wie Oleylalkohol, Octyldodecanol (Eutanol® G);

**Fettsäureester,** z.B. Isopropylfettsäureester (Palmitat, Myristat, Isostearat, Oleat), Decyl Oleate (Cetiol® V), Hexyl Laurate, C 12 - 95 Alkyl Benzoate (Finsolv® TN), Dicaprylyl Carbonate (Cetiol® CC), Diester wie Dibutyladipate (Cetiol® B), Propylenglykol Dipelargonate, verzweigte Fettsäureester wie PCL-liquid® (Cetearyl Octanoate) oder Gemische wie Cetiol® PGL (Hexyldecanol und Hexyldecyl Laurate)

**Fettalkoholether** wie Dicaprylyl Ether (Cetiol® OE)

**Polyolfettsäureester** wie Cetiol® HE (PEG-7 Glyceryl Cocoate)

**Triglyceride,** insbesondere mittelkettige (Neutralöle) wie Caprylic/Capric Triglyceride (Miglyol® 810, 812) sowie deren Polyolester wie Propylene Glycol Dicaprylate/ Dicaprate (Miglyol® 840)

**Natürliche Fette** und Öle wie Sonnenblumen-, Soja-, Pfirsichkem-, Aprikosenkern-, Traubenkern-, Ricinus-, Erdnuß-, Mandel-, Nerz-, Weizenkeim-, Avocadoöl.

**Natürliche flüssige Wachse,** z.B. Jojobaöl oder dessen Substitut Oleyl Erucate (Cetiol® J 600)

**Silikonöle und -wachse**, z.B. Polydimethylsiloxane wie Dow Corning Fluid® 200 (Dimethicone), Cyclomethylsiloxane wie Dow Corning Fluid® 345 (Cyclomethicone), Phenylmethylpolysiloxane wie Phenyl Dimethicone (Abil® AV 8853) oder Alkyl-Polymethylsiloxan-Copolymere wie Cetyl Dimethicone (Abil® Wax 9801), Stearyl Dimethicone (Abil® Wax 9800), Dialkoxydimethylpolysiloxane wie Stearoxy Dimethicone (Abil® Wax 2434), Behenoxy Dimethicone (Abil® Wax 2440)

Besonders bevorzugte Öl-Komponenten sind flüssige Paraffine, Fettsäureester wie Isopropylpalmitat oder -myristat, mittelkettige Triglyceride wie die vorgenannten Miglyole sowie die genannten natürlichen Fette und Öle, insbesondere Sonnenblumen-, Soja-, Pfirsichkern-, Aprikosenkernöl, insbesondere Mischungen hiervon, sowie Jojobaöl, dessen Mischungen mit den vorgenannten Komponenten, wobei jeweils ca. 1-40% an Einzelkomponente, bezogen auf die Gesamtmenge vorhanden sind oder Fettalkoholether oder Fettalkohole oder Mischungen hiervon oder hydrierte Öle wie Perhydrosqualen einzeln oder kombiniert.
Insbesondere bevorzugt werden die genannten flüssigen Paraffine und die genannten Trigyceride, auch in Kombination miteinander.
Ferner sind auch die o.g. Dioctylcyclohexane, Isohexadecane, insbesondere letztere, Silikonöle und -wachse besonders geeignet, vor allem auch Kombinationen hiervon und auch mit den vorgenannten Paraffinen und Trigyceriden. Diese können dann insbesondere auch mit den natürlichen Fetten, Ölen und Wachsen kombiniert werden.

Als vesikelbildende Lipide eignen sich insbesondere hierfür bekannte Stoffe (vgl. DE 42 05 548 C2), insbesondere Phospholipide wie Lecithin (Ei- oder Soja-Lecithin), z.B. Phosal® 50 SA (ca. 50 % Soja-Lecithin), Phosphatidylcholin, -serin oder -diethanolamin sowie deren Mischungen.

Weitere geeignete Komponenten zur Herstellung von Vesikeln sind Sphingolipide (z.B. Ceramide, Cerebroside, Sphingosin, Sphingomyelin), Phytosterole (im wesentlichen Gemische aus β-Sitosterol, Campesterol und Stigmasterol) sowie deren Derivate, insbesondere Ethoxylate wie Generol® 122 E 5 (PEG-5 Soybean Sterol), Gererol® R E5 (PEG-5 Rapeseed Sterol).

Ferner geeignet sind polyethoxylierte Fettalkohole sowie Fettsäuren mit vorzugsweise 1-4 EO mit einem HLB-Wert von 2 bis 6 wobei der lipophile Rest bevorzugt aus C₁₆ bis C₁₈-Alkylketten besteht, Polyglycerolalkylether, Glucosyldialkylether, Saccharosediester, Kollagenhydrolysatester, quartäre Ammoniumverbindungen und Poloxamere.

Besonders bevorzugte Vesikelbildner sind Phospholipide wie Ei- und/oder Soja-Lecithin, (Phosal® 50SA), Phosphatidylcholin, Ceramide, Phytosterole und ethoxilierte Derivate hiervon mit einem Ethoxilierungsgrad von 5 bis 16 und polyoxyethylierte Fettalkohole mit einem HLB-Wert von 2-6 oder Kombinationen hiervon, insbesondere von Phytosterolen der o.g. Art mit Ethoxylierungsprodukten hiervon.

Erfindungsgemäss ist es bevorzugt, wenn die ÖI- Zusammensetzung neben den Hauptkomponenten ÖI/EmulgatorlVesikelbildner als Zusatzstoffe Wirkstoffe, welche auch etherische Öle und Pflanzenextrakte umfassen, Konservierungsstoffe und Vitamine aufweist.

Als Vitamine eignen sich insbesondere Vitamin A, E, C und deren Derivate, z.B. Retinol, -acetat oder -palmitat, Carotinoide, Tocopherol oder -acetat, Ascorbylpalmitat.

Die Wirkstoffe sind bevorzugt ausgewählt aus etherischen Ölen und Terpenen, z.B. Rosmarinöl, Orangenöl, Lavendelöl, Limettenöl, Zimtöl, Geraniumöl, Zedemholzöl, Rosenholzöl, Baldrianöl, Ylang-Ylang Öl, Eucalyptusöl, Minzöl, Lemongrasöl, Zypressenöl, Niaouliöl, Fichtennadelöl, Kiefernnadelöl, Campher, Menthol.

Ferner können hier auch essentielle ungesättigte Fettsäuren und deren Ester, z.B. Linol- oder Linolensäure, Glyceryl Linoleate, Glyceryl Linolenate eingesetzt werden.

Alternativ oder zusätzlich zu den o.g. Wirkstoffen sind auch durchblutungsfördernde Stoffe, z.B. Nikotinsäurederivate wie Methyl- oder Tocopherylnikotinat, Alpha- und Betahydroxysäuren und deren Derivate, z.B. Salicysäure, Isopropylbenzylsalicylate, C12 - 13 Alkyl Lactate (Cosmacol® ELI) oder auch antiphlogistische und antibakterielle Substanzen wie Glycyrrhizinsäure oder Glycyrrhetinsäure und deren Derivate, z.B. Stearyl Glycyrrhetinate, Pantothensäurederivate, z.B. Panthenyltriacetat, Allantoin, Bisabolol, Azulene, z.B. Cham-Azulene oder Guaj-Azulen, Triclosan, Chlorhexidin-Derivate und/oder Antischuppenmittel, z. B. Climbazol oder Piroctone Olamine einsetzbar.

Daneben können in den erfindungsgemäßen Hautölen auch Repellentien wie N, N-Diethyl-m-toluamid oder Dimethylphtalat; oder antioxidativ sowie zellschützend wirkende Stoffe wie Flavonoide, z.B. Rutin, Ferulasäure und deren Ester oder Coenzym Q 10 als wirksame Zusatzstoffe eingesetzt werden.

Daneben sind auch Pflanzenextrakte, welche selbst auch Wirkstoffe sein können, einsetzbar, wie z.B.Äloe-Vera-Extract, Lindenblüten-Extract, Centella asiatica Extrakt, Efeublätter Extrakt, als wirksame Zusatzstoffe verwendbar.

Die genannten Komponenten können auch kombiniert werden, je nach gewünschtem Anwendungszweck. So sind insbesondere Kombinationen aus aromatherapeutisch wirkenden etherischen Ölen wie Rosmarin, Limetten- und/oder Lemongrasöl oder Baldrian-, Lavendel- und/oder Ylang-Ylang Öl oder durchblutungsfördernden Substanzen wie Rosmarinöl und Methylnikotinat bevorzugt.

Besonders bevorzugte Wirkstoffe sind ausgewählt aus etherischen Ölen und Terpenen, Pflanzenextrakten, durchblutungsfördernden Stoffen, antiphlogistischen und antibakteriellen Substanzen, Vitaminen, ungesättigten essentiellen Fettsäuren oder Mischungen hiervon.

Als weitere besonders geeignete Zusatzstoffe aus der Gruppe der Wirkstoffe, welche auch etherische Öle sein können wie oben beschrieben und auch gleichzeitig Parfümstoffe darstellen können, eignen sich insbesondere Minzöl, Limettenöl, Orangenöl, Wacholderöl, Baldrianöl, Eukalyptusöl, Thymianöl, Palmarosaöl, Rosmarinöl, Lavendelöl, Menthol, ingwer-, Lindenblüten-, Ringelblumen-, Algen-, Aloevera-, Echinacea-, Efeublätterextrakt, Hydroxyethylsalicylat, Methylsalicylat, Nikotinsäureester oder Kombinationen hiervon, wie z.B. Organgenöl, Lavendelöl, Palmarosaöl und Baldrianöl oder Wacholderöl, Hydroxyethylsalicylat und Methylnikotinat. Dabei kann je nach beabsichtigtem Wirkungseffekt, wie z.B. der Verbesserung der Hautstruktur, Erhöhung der Durchblutung, Entspannung, Aromatherapie u.ä. eine geeignete Kombination an Wirkstoffen zugesetzt werden. Gegebenenfalls können weitere Rückfetter wie Fettsäureglyceride und deren Ethoxylate, z. B. PEG-6 Caprylic/Capric Glycerides (Softigen® 767) hinzugefügt werden.

Es ist darüberhinaus bevorzugt, wenn die Hautöl-Zusammensetzung zusätzlich oder alternativ zu den drei obengenannten Zusatz-Varianten als Zusatzstoff solche, ausgewählt aus Antioxidantien, Parfümstoffe, Farbstoffe und/oder UV-Filter enthalten.

Die Antioxidantien können bevorzugt ausgewählt werden aus Butylhydroxytoluol, Butylhydroxyanisol, Ascorbylpalmitat, Tocopherol, evtl. in Kombination mit Synergisten wie in Controx® VP (Tocopherol, Lecithin, Ascorbyl Palmitate, Hydrogenated Palm Glycerides Citrate), Gallussäurealkylester wie Octyl-, Dodecyl- und Cetylgallat oder Kombinationen hiervon. Besonders bevorzugte Parfümstoffe sind neben den oben unter "Wirkstoffen" genannten etherischen Ölen auch handelsübliche Parfüm-Kompositionen.

Bevorzugte Farbstoffe sind z.B.. Patentblau, Amidoblau, Orange RGL, Cochenillerot, Chinolingelb, insbesondere in Verbindung mit einem geeigneten Lösungsvermittler. Dieser kann insbesondere ausgewählt werden aus Ethanol und Isopropanol, z.B. in mengen von 5-30%, bevorzugt 5-15%.
Besonders bevorzugte Farbstoffe sind Carotinoide z.B. alpha- oder beta-Carotin oder Azulene wie Cham-Azulen oder Guaj-Azulen.

Geeignete UV-Filter sind sind öllösliche UVB, UVA und Breitbandfilter der folgenden Art:
UV-B Filter: Zimtsäureester, z.B. Octyl Methoxycinnamate (Eusolex® 2292, Neo Heliopan® AV, Parsol® MCX), Isoamyl p-Methoxycinnamate (Neo Heliopan® Galanga) sowie 4-Methylbenzyliden Camphor (Eusolex® 6300), Paraaminobenzoesäure und -ester wie N, N-Dimethyl-4-aminobenzoesäure-2-ethylhexylester (Eusolex® 6007, Octyl Dimethyl PABA), Homomenthylsacilylat (Homosalate, Eusolex® HMS), Octyl Salicylate (Neo Heliopan® OS), Octocrylene (Neo Heliopan® 303), Butyl Methoxydibenzoylmethane (Eusolex® 9020);
UVA + UVB Filter für Breitbandabsorption wie Benzophenone-3 (Neo® Heliopan BB, Eusolex® 4360);
UV-A Filter wie Methyl Anthranilate (Neo Heliopan® MA)
Insbesondere bevorzugt sind Octyl- oder Isoamyl p- Methoxycinnamate, Octocrylene, 4-Methylbenzyliden Camphor, Homosalate und/oder Methyl Anthranilate und/oder Benzophenone-3.

Als Konservierungsstoffe kommen Iodopropynylbutylcarbamat, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, iso-Butylparaben, bevorzugt Methyl-oder Propylparaben oder Mischungen hiervon oder Climbazol oder geeignete Kombinationen der genannten Stoffe wie z.B. Methyl-, Propylparaben und Sorbinsäure in Frage.

Die erfindungsgemäßen emulgatorhaltigen Hautöl-Zusammensetzungen haben eine besondere vor allem selbstemulgierende Wirkung, insbesondere beim Auftragen auf die nasse Haut verbunden mit besserer Feuchtigkeitsspeicherung, besseres Einreiben in die Haut ohne unangenehmes Fettfilmgefühl, was insbesondere aus der erfindungsgemäßen Kombination von Ölkomponente und W/O-Emulgator resultiert.

Bevorzugte Kombinationen von Öl/Emulgator umfassen:
Paraffinöl (20-40 %) und/oder Miglyol 812 (20-30 %) und/oder Isopropylpalmitat (15-25%) ; oder auch zusätzlich hierzu Jojobaöl (1-5 %) und/oder Pfirsichkernöl (3-5 %) und als Emulgator Abil® EM 90 und/oder Abil ®WE 09.
Hierzu können die vorstehend genannten Vesikelbildner, insbesondere Phosal® 50SA sowie Zusatzstoffe insbesondere die genannten Kombinationen, und ganz besonders bevorzugt etherische Öle, Parfümöle und/oder durchblutungsfördernde Stoffe sowie Pflanzenextrakte und UV-Filter eingesetzt werden.

Soll das erfindungsgemäße Öl eine dünnflüssigere Konsistenz aufweisen , können auch 5-30 % , bevorzugt 5-15% Ethanol oder Isopropanol als Konsitenzmodulator, insbesondere Ethanol, zugesetzt werden. Diese können auch als Lösungsvermittler für andere Stoffe dienen.

Umgekehrt können als Konsistenzmodulatoren auch Verdicker und /oder Stoffe zur Verbesserung des Hautgefühls oder der Wasserfestigkeit hinzugefügt werden wie z.B. Polyethylene-Wachse mit einer Molmase von 1500 bis 20.000 wie Lunacera® PA Paste, Aerosil®, modifizierte Montmorillonite wie Miglyol® Gel B (Caprylic/Capric Triglyceride, Stearalkonium Hectorite, Propylene Carbonate), Aluminiumseifen sowie deren Modifikationen z.B. Aluminium/Magnesium Hydroxide Stearate, alkylierte Polyvinylpyrrolidone wie Antaron® V-216 (PVP/Hexadecene Copolymer) und Antaron® V-220 (PVP/Eicosene Copolymer).

Wenn bestimmte Zwecke beabsichtigt sind, z.B. Hautpflegeöl, Sportöl oder Sonnenschutzöl oder Massageöl, werden die Zusatzstoffe entsprechend ausgewählt, z.B. für Hautpflegeöl Vitamine, pflanzliche Öle und/oder Pflanzenextrakte,
für Sportöl durchblutungsfördernde Stoffe wie Methylnikotinat und/oder Hydroxyethylsalicylat und/oder etherische Öle, z.B. Rosmarinöl
für Sonnenschutzöl UV-A-, UV-B- und Breitbandfilter sowie Vitamine wie Vitamin E, Vitamin C
für Massageöl aromatherapeutisch und/oder durchblutungsfördernd wirkende etherische Öle wie Orangenöl, Lavendelöl, Palmarosaöl, Limettenöl, Lemongrasöl und/oder Vitamin A, Vitamin E
wobei zusätzlich Konservierungsmittel, Antioxidantien und/oder Parfümstoffe in den angegebenen Mengen vorhanden sein können.

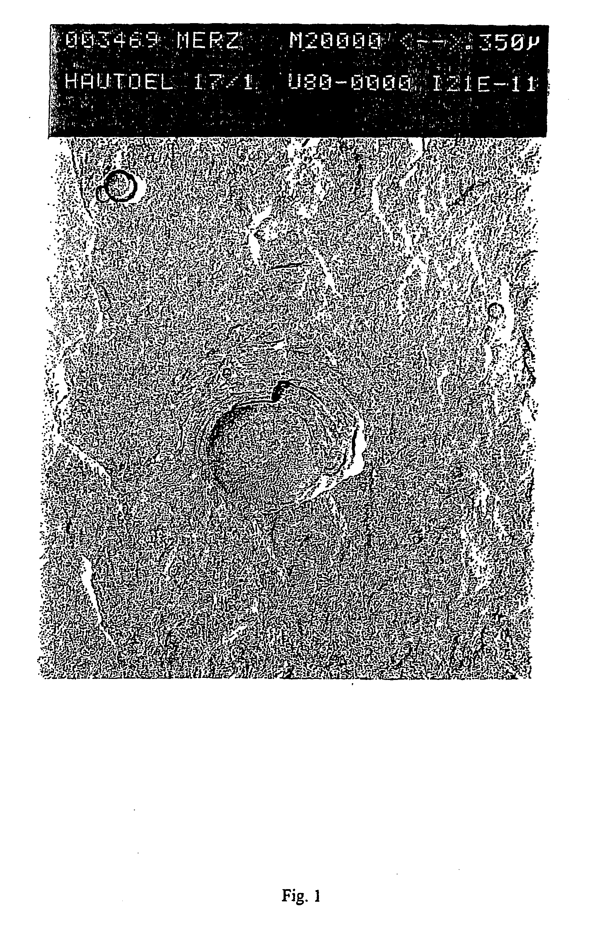

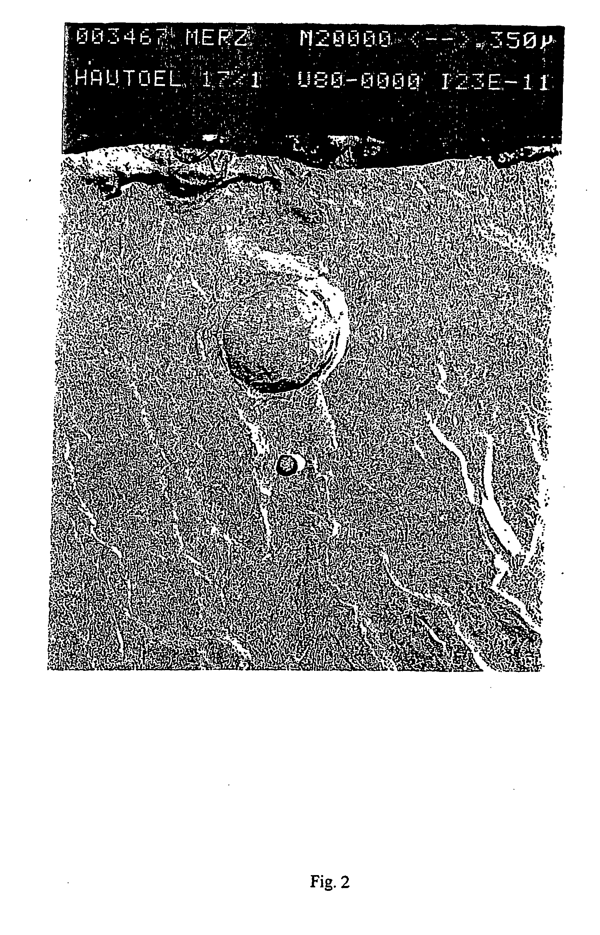

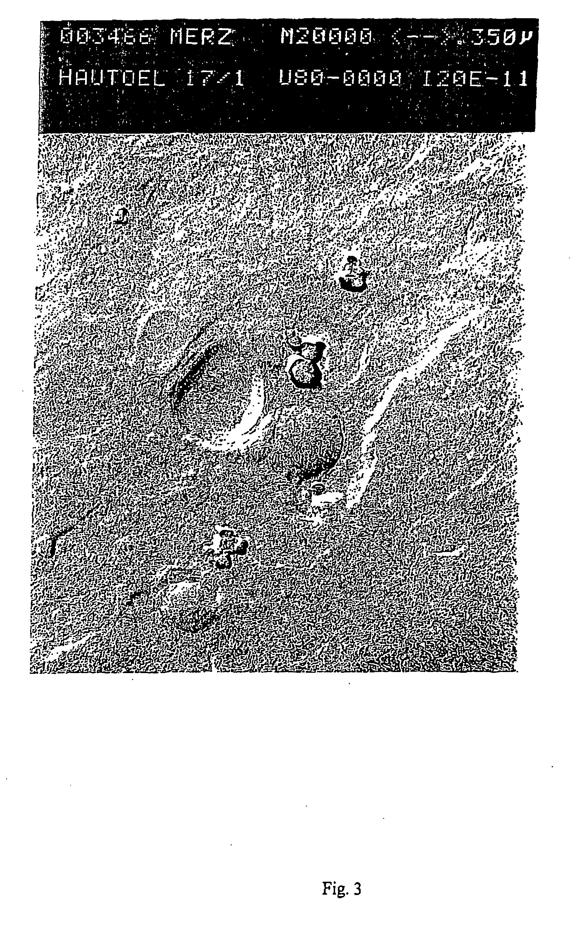

Die Erfindung wird anhand der nachfolgenden Beispiele 1-5 näher erläutert. Dabei zeigen die Fig. 1-3 die spontan entstandenen Liposomen nach Verdünnung mit Wasser im Verhältnis 1:1 bei 33 °C beim Auftragen der Zusammensetzungen gemäß Beispiel 1 im Transmissionselektronenmikroskop nach Gefrierbruch.

Die erfindungsgemässen Zusammensetzungen wurden dabei hergestellt, indem die flüssigen Öle (wenn vorhanden) in einem geeigneten Gefäss bei Raumtemperatur homogen verrührt, gegebenenfalls erwärmt wurden auf 40-90°C, um gegebenenfalls vorhandene feste Wachse oder Fette zu schmelzen. Danach wurden der oder die Emulgatoren eingearbeitet.
Nach dem Abkühlen auf Raumtemperatur, insbesondere 25°C, wurden sofern vorhanden, die Zusatzstoffe eingearbeitet und homogen verrührt.
Gegebenenfalls können feste Komponenten auch vorab in geeigneter Weise erwärmt werden, und sodann die nicht festen Bestandteile bei geeigneter Temperatur(25-50°C, insbesondere 25-40°C) hinzugefügt werden.

**Beispiel 1 Hautöl**

| **Rohstoffe** | **100 % Rezeptur** |
|---|---|
| Miglyol 812 | 36,80 |
| Pfirsichkernöl | 3,00 |
| Jojobaöl | 1,00 |
| Tocopherolacetat | 1,10 |
| Paraffinöl, dickflüssig | 28,40 |
| Isopropylpalmitat | 25,00 |
| Abil® EM 90: Cetyl Dimethicone Copolyol | 2,00 |
| Phosal® 50 SA:50% Lecithin | 2,00 |
| Parfümöl | 0,70 |
| **SUMME** | **100,00** |

### Bemerkungen:

Abil® EM 90: Polysiloxan-Polyalkyl-Polyether-Copolymer

**Beispiel 2 Hautöl**

| **Rohstoffe** | **100 % Rezeptur** |
|---|---|
| Miglyol 812 | 36,12 |
| Pfirsichkernöl | 4,00 |
| Tocopherolacetat | 1,10 |
| Paraffinöl, dickflüssig | 28,40 |
| Isopropylpalmitat | 25,00 |
| Abil® EM 90 | 2,00 |
| Phosal 50 SA:50% Lecithin | 2,00 |
| Baldrianöl | 0,08 |
| Zedernholzöl | 0,20 |
| Rosenholzöl | 0,10 |
| Parfümöl | 1,00 |
| **SUMME** | **100,00** |

**Beispiel 3 Sonnenhautschutzspray auf Ölbasis**

| **Rohstoffe** | **100 % Rezeptur** |
|---|---|
| Paraffinöl, dünnflüssig | 50,50 |
| Finsolv TN: C12-C15-Alkylbenzoate | 10,00 |
| Cetiol® OE: Dicaprylylether | 10,00 |
| Miglyol 812 | 20,00 |
| Abil® EM 90 | 2,00 |
| Phosal 50 SA:50% Lecithin | 2,00 |
| UV-Filter: Neo Heliopan AV: Octyl-Methoxycinnamate | 5,00 |
| Parfümöl | 0,50 |
| | |
| **SUMME** | **100,00** |

**Beispiel 4 Hautöl**

| **Rohstoffe** | **100 % Rezeptur** |
|---|---|
| Miglyol 812 | 37,00 |
| Pfirsichkernöl | 3,00 |
| Jojobaöl | 1,00 |
| Tocopherolacetat | 1,10 |
| Paraffinöl, dickflüssig | 28,40 |
| Isopropylpalmitat | 25,00 |
| Dehymuls® PGPH: Polyglyceryl-2-dipolyhydroxistearate | 2,00 |
| Phosal® 50 SA: 50% Lecithin | 2,00 |
| Parfümöl | 0,50 |
| **SUMME** | **100,00** |

**Beispiel 5 Hautöl**

| **Rohstoffe** | **100 % Rezeptur** |
|---|---|
| Miglyol 812 | 37,00 |
| Pfirsichkernöl | 3,00 |
| Tocopherolacetat | 1,10 |
| Paraffinöl, dickflüssig | 28,40 |
| Isopropylpalmitat | 26,00 |
| Isolan® PDI: Diisostearoyl Polyglyceryl-3 Diisostearate | 2,00 |
| Phosal® 50 SA: 50% Lecithin | 2,00 |
| Parfümöl | 0,50 |
| **SUMME** | **100,00** |

## Patentansprüche

1. Vesikelbildendes Hautöl, enthaltend eine oder mehrere öllösliche Komponenten, einen oder mehrere W/O-Emulgatoren mit einem HLB-Wert von 2-5,9, ausgewählt aus ethoxylierten Fettsäuren, ethoxylierten Triglyceriden, (Poly)glycerylestern, Glucose-Estem, Polysiloxan-Copolymeren, Siloxan-Derivate haltigen Emulgator-Gemischen oder Mischungen hiervon, und ein oder mehrere vesikelbildende Lipide, sowie gegebenenfalls einen oder mehrere Zusatzstoffe, ausgewählt aus Wirkstoffen, Antioxidantien, Parfümstoffen, Konservierungsmitteln, UV-Filtern, Vitaminen, Farbstoffen, Konsistenzmodulatoren, Lösungsvermittlern, mit Ausnahme von Zusammensetzungen, enthaltend Cholesterol und/oder Wollwachsalkohol.

2. Hautöl gemäss Anspruch 1 **dadurch gekennzeichnet, dass** die öllöslichen Komponente(n) in einer Menge von 50-99 %, der oder die Emulgatoren in einer Menge von 0,5-10 % , das oder die vesikelbildenden Lipide in einer Menge von 0,01-10% und der oder die Zusatzstoffe in einer Menge von 0-40 % insgesamt vorhanden sind.

3. Hautöl gemäß einem der Ansprüche 1 oder 2, **dadurch gekennzeichnet, dass** die Ölkomponente/n ausgewählt ist / sind aus Kohlenwasserstoffen, Fettalkoholen, Fettsäureestern, Fettalkoholethern, Polyolfettsäureestem, Triglyceriden, natürlichen Fetten ,Ölen oder natürlichen flüssigen Wachsen oder Silikonölen oder -wachsen oder Mischungen hiervon.

4. Hautöl gemäß einem der Ansprüche 1 bis 3, **dadurch gekennzeichnet, dass** die Ölkomponente ausgewählt ist aus flüssigem Paraffin, Isopropylpalmitat, mittelkettigen Triglyceriden, Sonnenblumen-, Soja-, Pfirsichkem-, Aprikosenöl, Jojobaöl, Silikonöle oder Mischungen der vorgenannten Komponenten.

5. Hautöl gemäss einem der Ansprüche 1-4, **dadurch gekennzeichnet, dass** der oder die W/O-Emulgatoren in einer Menge von 1,0-8% vorhanden sind.

6. Hautöl gemäß einem der Ansprüche 1-5, **dadurch gekennzeichnet, dass** der oder die W/O-Emulgatoren einen HLB-Wert von 3,5-5,5 aufweisen.

7. Hautöl gemäß einem der Ansprüche 1-6, **dadurch gekennzeichnet, dass** als Emulgator Cetyl Dimethicone Copolyol oder Cetyl Oimethicone Copolyol im Gemisch mit Polyglyceryl-4 Isostearat und Hexyl Laurat vorhanden ist.

8. Hautöl gemäß einem der Ansprüche 1-7, **dadurch gekennzeichnet, dass** das vesikelbildende Lipid ausgewählt ist aus Phospholipiden wie Ei- und/oder SojaLecithin, Phosphatidylcholin, Ceramiden, Phytosterolen und ethoxilierten Derivaten hiervon mit einem Ethoxilierungsgrad von 5 bis 16 und polyoxyethylierten Fettalkoholen mit einem HLB-Wert von 2-6 oder Kombinationen hiervon, insbesondere von Phytosterolen der o.g. Art mit Ethoxylierungsprodukten hiervon.

9. Hautöl gemäss einem der Ansprüche 1-8, **dadurch gekennzeichnet, dass** das Hautöl als Zusatzstoffe Wirkstoffe, Konservierungsstoffe und Vitamine aufweist.

10. Hautöl gemäss einem der Ansprüche 1-9, **dadurch gekennzeichnet, dass** das Hautöl als Zusatzstoffe solche, ausgewählt aus Antioxidantien, Parfümstoffen, Farbstoffen, UV-Filtem aufweist.

11. Hautöl gemäss Anspruch 9 oder 10, **dadurch gekennzeichnet, dass** die Wirkstoffe ausgewählt sind aus etherischen Ölen und Terpenen, Pflanzenextrakten, durchblutungsfördemden Stoffen, antiphlogistischen und antibakteriellen Substanzen oder Mischungen hiervon.

12. Hautöl gemäß einem der Ansprüche 1-11, **dadurch gekennzeichnet, dass** das/die Parfüms handelsübliche Parfümöl- Komponsitionen darstellen, das/die Konservierungsmittel ausgewählt sind aus Methyl- oder Propylparaben oder Mischungen hiervon und/oder Sorbinsäure, die Antioxidantien ausgewählt sind aus Butylhydroxytoluol, Butylhydroxyanisol, Ascorbylpalmitat, Tocopherol oder deren Gemische mit Synergisten, die Vitamine ausgewählt sind aus Vitamin E, Vitamin A, Vitamin C und deren Derivaten.

13. Hautöl gemäss einem der Ansprüche 1-12, **dadurch gekennzeichnet, dass** die Ölkomponente/n in einer Menge von 85-97%, der/die W/O-Emulgatoren in einer Menge von 2-6% das oder die vesikelbildenden Lipide in einer Menge von 0,5-5% und die Zusatzstoffe in einer Menge von 1-15% vorhanden sind.

14. Hautöl gemäss einem der Ansprüche 1-13, **dadurch gekennzeichnet, dass** als Lösungsvermittler Ethanol vorhanden ist.

15. Verfahren zur Herstellung von Hautölen gemäß einem der Ansprüche 1-14, **dadurch gekennzeichnet, dass** man das oder die Ölkomponenten bei Raumtemperatur mischt und ggf. auf 40-90°C erwärmt, den oder die Emulgatoren einarbeitet, sodann auf Raumtemperatur abkühlt und anschließend den oder die Zusatzstoffe inkorporiert.

16. Nichttherapeutische Verwendung von Hautölen gemäß einem der Ansprüche 1-14 als Hautpflegeöl, Hautschutzöl, Sportöl, Massageöl oder Sonnenschutzöl.

## Claims

1. Vesicle-forming skin oil comprising one or more oil-soluble components and one or more W/O emulsifiers with an HLB value of 2-5,9 chosen from ethoxylated fatty acids, ethoxylated triglycerides, (poly)glyceryl esters, glucose esters, polysiloxane copolymers, emulsifier mixtures containing siloxane derivatives, or mixtures thereof, and one or more vesicle-forming lipids, and optionally one or more additives, chosen from active ingredients, antioxidants, perfume substances, preservatives, UV filters, vitamins, dyes, consistency modulators, solubilisers, with the exception of compositions comprising cholesterol and/or wool wax alcohol.

2. Skin oil according to claim 1, **characterized in that** the oil soluble component(s) is/are present in an amount of 50-99%, the emulsifier(s) is / are present in an ampount of 0,5-10%, the vesicle-forming lipid(s) is /are present in an amount of 0,01-10% and the additive(s) is / are present in an amount of 0-40%.

3. Skin oil according to any one of claim 1 or 2, **characterized in that** the oil component(s) is / are chosen from hydrocarbons, fatty alcohols, fatty acid esters, fatty alcohol ethers, polyol fatty acid esters, triglycerides, natural fats, oils or natural liquid waxes or silicone oils or silicone waxes or mixtures thereof.

4. Skin oil according to any one of claims 1 to 3, **characterized in that** the oil component is chosen from liquid paraffin, isopropyl palmitate, medium-chain triglycerides, sunflower oil, soybean oil, peach kernel oil, apricot oil, jojoba oil, silicone oils or mixtures of the above-mentioned components.

5. Skin oil according to any one of claims 1 to 4, **characterized in that** the W/O emulsifier(s) is/are present in an amount from 1,0-8%.

6. Skin oil according to any one of claims 1 to 5, **characterized in that** the W/O-emulsifier(s) has/have an HLB value of 3.5-5.5.

7. Skin oil according to any one of claims 1 to 6, **characterized in that** cetyl dimethicone copolyol or cetyl dimethicone copolyol in a mixture with polyglyceryl-4- isostearate and hexyl laurate is present as the emulsifier.

8. Skin oil according to any of claims 1 to 7 **characterized in that** the vesicle forming lipid is chosen from phospholipids, such as egg and / or soybean lecithin, phosphatidylcholine, ceramides, phytosterols and ethoxylated derivatives thereof with a degree of ethoxylation of 5 to 16 and polyoxyethylated fatty alcohols with an HLB value of 2-6 or combinations thereof, in particular of phytosterols of the above mentioned type with ethoxylation products thereof.

9. Skin oil according to any one of claims 1 to 8, **characterized in that** the skin oil has active ingredients, preservatives and vitamins as additives.

10. Skin oil according to any one of claims 1 to 9, **characterised in that** the skin oil has, as additives, those chosen from antioxidants, perfume substances, dyes, UV filters.

11. Skin oil according to either claim 9 or claim 10, **characterized in that** the active ingredients are chosen from essential oils and terpenes, plant extracts, circulation-promoting substances, antiphlogistics and antibacterial substances or mixtures thereof.

12. Skin oil according to any one of claims 1 to 11, **characterized in that** the perfume(s) is/are conventional commercial perfume oil compositions, the preservative(s) is/are chosen from methylparaben or propylparaben or mixtures thereof and / or sorbic acid, the antioxidants are chosen from butylhydroxytoluene, butylhydroxyanisole, ascorbyl palmitate, tocopherol or mixtures thereof with synergistic agents, the vitamins are chosen from vitamin E, vitamin A, vitamin C and derivatives thereof.

13. Skin oil according to any one of claims 1 to 12, **characterized in that** the oil component(s) is / are present in an amount of 85-97%, the W/O emulsifier(s) is / are present in an amount of 2-6%, the vesicle-forming lipid(s) is/are present in an amount of 0,5-5% and the additives are present in an amount of 1-15%.

14. Skin oil according to one any of claims 1 to 13 **characterized in that** ethanol is present as solubilizer.

15. Method for producing skin oils according to any one of claims 1 to 14, **characterized in that** the oil component(s) is/are mixed at room temperature and optionally heated to 40-90 °C, the emulsifier(s) is/are incorporated, then the mixture is cooled to room temperature and then the additive(s) is/are incorporated.

16. Non therapeutic use of skin oils according to any one of claims 1 to 14 as skincare oil, skin protection oil, sport oil, massage oil or sunscreen oil.

## Revendications

1. Huile à appliquer sur la peau, formant des vésicules, contenant un ou plusieurs composants liposolubles et un ou plusieurs émulsifiants eau/huile avec une valeur HLB de 2 à 5,9, choisis parmi les acides gras éthoxylés, les triglycérides éthoxylés, les esters de (poly)glycéryle, les esters du glucose, les copolymeres de polysiloxane, des mélanges d' émulsifiants contenant des dérivés du siloxane, ou des mélanges de ceux-ci,
et un ou plusieurs lipides formant des vésicules, de même que, le cas échéant une ou plusieurs substances additionnelles, choisies parmi des principes actifs, des antioxydants, des parfums, des agents de conservation, des filtres d'UV, des vitamines, des colorants, des modulateurs de consistance, des médiateurs de dissolution, à l'exception des compositions contenant du cholestérol et/ou de l'alcool de cire de laine.

2. Huile à appliquer sur la peau selon la revendication 1, **caractérisée en ce que** le ou les composants liposolubles sont présents en tout en une quantité de 50 à 99 %, **en ce que** le ou les émulsifiants sont présents en tout en une quantité de 0,5 à 10 %, **en ce que** les lipides formant des vésicules sont présents en tout en une quantité de 0,01 à 10 % et **en ce que** la ou les substances additionnelles sont présentes en tout en une quantité de 0 à 40 %.

3. Huile à appliquer sur la peau selon l'une des revendications 1 ou 2, **caractérisée en ce que** le ou les composants d'huile sont choisis parmi les hydrocarbures, les alcools gras, les esters d'acides gras, les éthers d'alcools gras, les esters de polyols et d'acides gras, les triglycérides, les graisses naturelles, les huiles ou les cires liquides naturelles ou les huiles ou les cires de silicones, ou des mélanges de ceux-ci

4. Huile à appliquer sur la peau selon l'une des revendications 1 à 3, **caractérisée en ce que** le composant d'huile est choisi parmi la paraffine liquide, le palmitate d'isopropyle, les triglycérides à chaîne moyennes, les huiles de tournesol, de soja, d'amande de pêche, d'abricot, l'huile de jujube, les huiles de silicones ou des mélanges des composants susmentionnés.

5. Huile à appliquer sur la peau selon l'une des revendications 1 à 4, **caractérisée en ce que** le ou les émulsionnants eau/huile sont présents en une quantité de 1,0 à 8 %.

6. Huile à appliquer sur la peau selon l'une des revendications 1 à 5, **caractérisée en ce que** le ou les émulsionnants eau/huile présentent une valeur HLB de 3,5 à 5,5.

7. Huile à appliquer sur la peau selon l'une des revendications 1 à 6, **caractérisée en ce que** du copolyol de cétyl diméticone ou du copolyol de cétyl diméticone en mélange avec de l'isostéarate de polyglycéryle-4 et du laurate d'hexyle sont présents comme émulsionnants.

8. Huile à appliquer sur la peau selon l'une des revendications 1 à 7, **caractérisée en ce que** le lipide formant des vésicules est choisi parmi les phospholipides comme la lécithine de l'oeuf et/ou du soja, la phosphatidylcholine, les céramides, les phytostérols et les dérivés éthoxylés de ceux-ci avec un degré d'éthoxylation de 5 à 16 et des alcools gras polyoxyéthylés avec une valeur HLB de 2 à 6, ou des combinaisons de ceux-ci, en particulier de phytostérols de la nature définie ci-dessus avec des produits d'éthoxylation.

9. Huile à appliquer sur la peau selon l'une des revendications 1 à 8, **caractérisée en ce que** l'huile à appliquer sur la peau comporte comme substances additionnelles des principes actifs, des agents de conservation et des vitamines.

10. Huile à appliquer sur la peau selon l'une des revendications 1 à 9, **caractérisée en ce que** l'huile à appliquer sur la peau comporte comme substances additionnelles des substances qui sont choisies parmi des antioxydants, des parfums, des colorants, des filtres d'UV.

11. Huile à appliquer sur la peau selon la revendication 9 ou 10, **caractérisée en ce que** les principes actifs sont choisis parmi les huiles éthérées et les terpènes, les extraits de plantes, les substances vasodilatatrices, les substances antiphlogistiques et antibactériennes ou des mélanges de ceux-ci.

12. Huile à appliquer sur la peau selon l'une des revendications 1 à 11, **caractérisée en ce que** le ou les parfums représentent des compositions d'huiles de parfum du commerce, **en ce que** le ou les agents de conservation sont choisis parmi le méthyl- ou le propylparabène ou des mélanges de ceux-ci, **en ce que** les antioxydants sont choisis parmi le butylhydroxytoluène, le butylhydroxyanisol, le palmitate d'ascorbyle, le tocophérol ou leurs mélanges avec des synergistes, **en ce que** les vitamines sont choisies parmi la vitamine E, la vitamine A, la vitamine C et leurs dérivés.

13. Huile à appliquer sur la peau selon l'une des revendications 1 à 12, **caractérisée en ce que** le ou les composants d'huile sont présents dans une quantité de 85 à 97 %, **en ce que** le ou les émulsifiants eau/huile sont présents dans une quantité de 2 à 6 %, **en ce que** le ou les lipides formant des vésicules sont présents dans une quantité de 0,5 à 5 % et **en ce que** les substances additionnelles sont présentes dans une quantité de 1 à 15 %.

14. Huile à appliquer sur la peau selon l'une des revendications 1 à 13, **caractérisée en ce que** de l'éthanol est présent comme médiateur de dissolution.

15. Procédé de production d'huiles à appliquer sur la peau selon l'une des revendications 1 à 14, **caractérisé en ce que** l'on mélange le ou les composants d'huile à la température ambiante, et qu'on les réchauffe le cas échéant à 40 à 90 °C, que l'on y incorpore les émulsionnants, qu'on les refroidisse alors à la température ambiante et que l'on y incorpore ensuite la ou les substances additionnelles.

16. Utilisation non thérapeutique des huiles à appliquer sur la peau selon l'une des revendications 1 à 14 comme huile de soins de la peau, huile de protection de la peau, huile de sport, huile de massage ou huile solaire.
